# EUROPÄISCHE PATENTANMELDUNG

(11) **EP 1 062 968 A2**
(43) Veröffentlichungstag der Anmeldung: **27.12.2000**
(21) Anmeldenummer: 00109840.9
(22) Anmeldetag: 10.05.2000
(51) Int. Cl.: A61N 1/05

(54) **Vorrichtung zum Herausziehen eines ein längliches Innenlumen aufweisenden Gegenstandes aus seiner Verankerung in einem Körper**

(30) Priorität: 24.06.1999 DE 19928901
(71) Anmelder: Vascomed Institut für Kathetertechnologie GmbH, 79576 Weil am Rhein (DE)
(72) Erfinder: Reinhardt, Jörg, 79639 Grenzach-Wyhlen (DE)
(74) Vertreter: RACKETTE Partnerschaft Patentanwälte

(57) **Zusammenfassung**

Eine Extraktionsvorrichtung (1) für Herzschrittmacher- oder Defibrillator-Elektroden verfügt über ein Verankerungsteil (4), das mit Hilfe einer Betätigungseinrichtung (20) von einer Verschiebegestalt in eine aufgeweitete Verriegelungsgestalt verformbar ist. Das Verankerungsteil (4) besteht aus einem Rohrabschnitt (12) mit mehreren in axialer Richtung versetzten Wandausschnitten (13), zwischen denen Führungsringe (14) verbleiben, die durch rinnenförmige Stege (15) verbunden sind. Durch Ausüben einer Zugkraft an einem Steuerdraht (8) mit Hilfe der Betätigungseinrichtung (20) werden der Rohrabschnitt (12) gestaucht und die rinnenförmigen Stege (15) radial verformt, so daß sie mit dem Innenlumen der Spiralwendel eines Herzschrittmacher- oder Defibrillator-Elektrodenkabels in Eingriff gelangen und infolge eines Spiralversatzes einen hohe Auszugskräfte gestattenden Formschluß bewirken.

## Beschreibung

Die Erfindung betrifft eine Vorrichtung zum Herausziehen eines ein längliches Innenlumen aufweisenden Gegenstandes aus seiner Verankerung in einem Körper, insbesondere einer Herzschrittmacher- oder Defibrillator-Elektrode, mit einem eine Spiralwendel aufweisenden Elektrodenkabel, wobei die Vorrichtung ein in das Innenlumen des Gegenstandes einführbares Verankerungsteil, das mit Hilfe einer Betätigungseinrichtung aktivierbar ist, und ein Rohr aufweist, durch das sich ein mit der Betätigungseinrichtung verbundener Steuerdraht erstreckt, durch den bei einer Relativbewegung zwischen dem Rohr und dem Steuerdraht auf das Verankerungsteil eine axial einwirkende Kraft einleitbar ist, durch die das Verankerungsteil zwischen einer Verschiebegestalt und einer aufgeweiteten Verriegelungsgestalt verformbar ist.

Eine derartige Vorrichtung ist in der US 5,632,749 A beschrieben. Bei einer der vorbekannten Ausführungsformen ist das Verankerungsteil als geschlitzte Hülse ausgebildet, die durch die Relativbewegung zwischen dem Rohr und dem Steuerdraht aktivierbar ist. Die Hülse ist entlang ihrer Längsachse geschlitzt und so ausgebildet, daß sie zum Aktivieren der Vorrichtung in ihrem Durchmesser vergrößert werden kann. Dazu sind am distalen Ende des Rohres sowie am distalen Ende des Steuerdrahtes aufeinanderzuweisende Konusflächen vorgesehen, die mit der geschlitzten Hülse durch Ziehen am Steuerdraht in Eingriff gebracht werden können, so daß sich die geschlitzte Hülse aufweitet und entlang einer Vielzahl von Windungen der Spiralwendel des Elektrodenkabels angedrückt wird, um hierdurch einen Kraftschluß zu erzielen.

Nachteilig bei dieser Vorrichtung ist es, daß zur Erzielung eines ausreichenden Kraftschlusses hohe axiale Kräfte auf die geschlitzte Hülse durch Ziehen am Steuerdraht und Gegenhalten mit dem Rohr erzeugt werden müssen. Ein weiterer Nachteil besteht darin, daß nur eine verhältnismäßig geringe Vergrößerung des Durchmessers der geschlitzten Hülse möglich ist, weshalb je nach dem Durchmesser des Innenlumens der Spiralwendel der Herzschrittmacher- oder Defibrillator-Elektrode unterschiedlich dimensionierte Extraktionsvorrichtungen bereitgehalten werden müssen.

Eine weitere aus der US 5,632,749 A bekannte Extraktionsvorrichtung verfügt über einen Schlauch, der in der Nähe seines distalen Endes mehrere auf dem Umfang des Schlauches verteilte, sich axial erstreckende parallelverlaufende Durchtrennungen aufweist. Zwischen den Durchtrennungen werden auf diese Weise mehrere in Umfangsrichtung unmittelbar benachbarte Streifen gebildet. Der Steuerdraht der in Fig. 12 der US 5,632,749 A dargestellten Extraktionsvorrichtung ist mit dem distalen Ende des Schlauches verbunden. Bei einem Zug am Steuerdraht wird das vordere Schlauchende gestaucht, wobei sich die zwischen den Einschnitten gebildeten Streifen nach außen verformen und gegen die Spiralwendel angedrückt werden.

Die mit dieser Vorrichtung erzielbaren Auszugskräfte sind äußerst gering, da der durch die radial ausgebeulten Streifen erzeugbare Reibschluß infolge der geringen Kontaktfläche der ausgebeulten Streifen mit der Spiralwendel auf eine sehr kleine Fläche begrenzt ist.

Ausgehend von dem oben erörterten Stand der Technik liegt der Erfindung die Aufgabe zugrunde, eine Extraktionsvorrichtung, insbesondere eine Extraktionsvorrichtung für einspiralige oder mehrspiralige Herzschrittmacher- oder Defibrillator-Elektroden zu schaffen, die weitgehend unabhängig vom Durchmesser des Innenlumens zuverlässig einsetzbar ist und hohe Auszugskräfte zu übertragen gestattet.

Diese Aufgabe wird bei einer Vorrichtung der eingangs genannten Art dadurch gelöst, daß das Verankerungsteil in seiner Verriegelungsgestalt über mehrere in axialer Richtung gegeneinander versetzte radiale Aufweitungen verfügt.

Infolge der in axialer Richtung gegeneinander versetzten radialen Aufweitungen erfolgt eine Deformierung der Spiralwendel durch einen Spiralversatz benachbarter Spiralen, so daß neben einer reibschlüssigen Verbindung des Verankerungsteils mit der Spiralwendel eine hohe Auszugskräfte erlaubende formschlüssige Verbindung gebildet wird. Dieser Effekt ist besonders bei mehrspiraligen Herzschrittmacher- oder Defibrillator-Elektroden bereits bei geringen radialen Kräften des Verankerungsteils bemerkbar.

Bei einem bevorzugten Ausführungsbeispiel besteht das Verankerungsteil aus einem dünnwandigen metallischen Rohrabschnitt, der mit sich axial erstreckenden Wandeinschnitten versehen ist, die im axialen Abstand voneinander angeordnet sind. Dabei können die Wandeinschnitte abwechselnd auf sich gegenüberliegenden Seiten der Rohrwandung ausgebildet sein. Alternativ ist es auch möglich, daß benachbarte Wandeinschnitte in Rohrumfangsrichtung um jeweils ein Viertel des Rohrumfangs versetzt angeordnet sind.

Die Anordnung der Wandeinschnitte kann auch so erfolgen, daß zwischen benachbarten Wandeinschnitten in radialer Richtung leicht verformbare Stege verbleiben. Dabei kann die Anordnung auch so getroffen sein, daß zusätzlich zu den in axialer Richtung gegeneinander versetzten Stegen auch sich radial gegenüberliegende Stege oder entlang der Umfangsrichtung mehrere Stege vorgesehen sind, die in axialer Richtung gegeneinander nicht versetzt sind.

Die Länge der Wandeinschnitte zur Erzeugung der nicht versetzten beziehungsweise der versetzten Stege beträgt ein Vielfaches des Rohrdurchmessers, beispielsweise das Dreifache des Rohrdurchmessers.

Die Wandeinschnitte können auch als breitere Wandausschnitte ausgebildet sein. Insbesondere können sich die Wandausschnitte so weit in den Rohrmantel oder die Rohrwandung erstrecken, daß von dem ursprünglichen Rohr nur noch eine Rinne übrigbleibt. Wenn die Höhe dieser Rinne wesentlich kleiner als der Rohrhalbmesser ist, ergibt sich eine besonders gute Verformbarkeit.

Bei einem zweckmäßigen Ausführungsbeispiel ist es vorgesehen, daß die Wandausschnitte an ihren axialen Enden mit der Rohrlängsachse einen Winkel von etwa 30 Grad bis 90 Grad, vorzugsweise 45 Grad bilden.

Das distale Ende des Steuerdrahtes ist bei einem bevorzugten Ausführungsbeispiel fest mit dem distalen Ende des das Verankerungsteil bildenden Rohrabschnitts verbunden. Auf diese Weise kann der das Verankerungsteil bildende Rohrabschnitt nicht nur auf Stauchung sondern auch auf Streckung zwecks Deformation des Verankerungsteils beansprucht werden.

Bei einem anderen Ausführungsbeispiel ist das distale Ende des Steuerdrahtes mit einem Anschlagkopf versehen, der gegen das distale Ende des den Verankerungsteil bildenden Rohrabschnittes anschlagbar ist. Alternativ ist es möglich, das distale Ende des Rohres als verformbaren Rohrabschnitt auszubilden, der einstückig mit dem Rohr verbunden ist, oder aber das Verankerungsteil beziehungsweise den Rohrabschnitt als vom Rohr getrenntes beziehungsweise trennbares selbständiges Teil vorzusehen.

Nachfolgend werden Ausführungsbeispiele der Erfindung anhand der Zeichnungen näher erläutert. Es zeigen:
- Fig. 1: eine Ausführungsform der Erfindung in Gestalt einer Vorrichtung zum Entfernen transvenös implantierter Herzschrittmacher- oder Defibrillator-Elektroden in einem Längsschnitt, wobei das links aus der dargestellten Betätigungseinrichtung herausragende Rohr stark verkürzt und abgeschnitten dargestellt ist,
- Fig. 2: das Verankerungsteil der Vorrichtung im Innenlumen der Spiralwendel eines Elektrodenkabels im Längsschnitt in einer gegenüber Fig. 1 vergrößerten Darstellung,
- Fig. 3: eine der Fig. 2 entsprechende Darstellung des Verankerungsteil, nachdem dieses in die Verriegelungsgestalt überführt worden ist und in der Spiralwendel des Elektrodenkabels aufgrund der Deformation des Verankerungsteils mehrere Spiralversätze erzeugt hat,
- Fig. 4: eine gegenüber den Fig. 1 bis 3 abgewandelte Ausführungsform der Erfindung, bei der ein gegenüber dem Verankerungsteil beweglicher Anschlagkopf vorgesehen ist, in einer Draufsicht,
- Fig. 5: eine Seitenansicht auf das in Fig. 4 in Draufsicht dargestellte Verankerungsteil zusammen mit einem das Verankerungsteil durchquerenden Steuerdraht und dem gegenüber dem Verankerungsteil beweglichen Anschlagkopf,
- Fig. 6: eine der Fig. 4 entsprechende Darstellung einer abgewandelten Ausführungsform des Verankerungsteil, bei dem benachbarte Wandausschnitte im Gegensatz zu den Darstellungen in den Fig. 4 und 5 nicht um 180 Grad sondern um 90 Grad gegeneinander versetzt sind und
- Fig. 7: eine der Fig. 6 entsprechende Darstellung, bei der das Verankerungsteil gemäß Fig. 6 um 90 Grad um seine Längsachse verdreht worden ist.

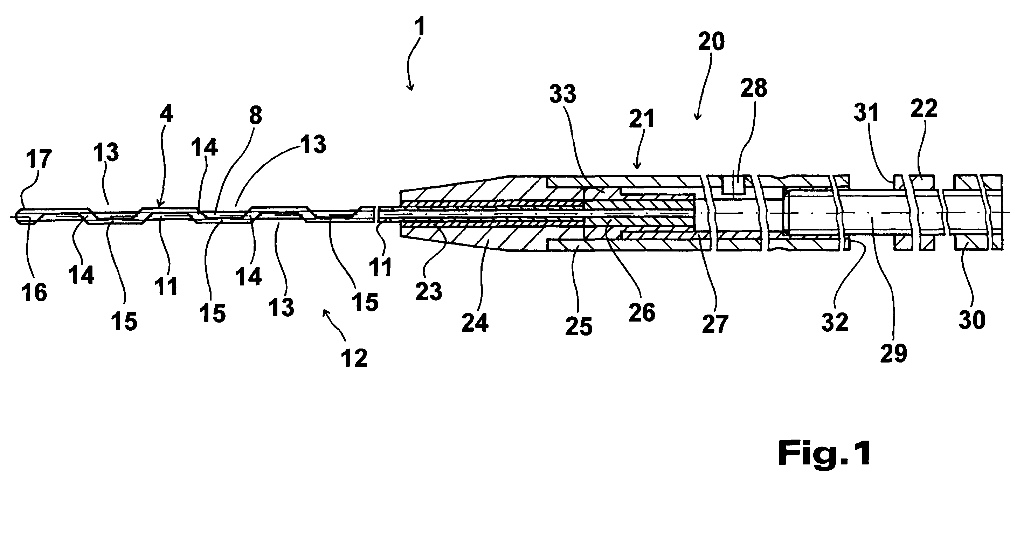

Die in Fig. 1 dargestellte Vorrichtung zum Herausziehen eines ein längliches Innenlumen aufweisenden Gegenstandes aus seiner Verankerung in einem Körper ist eine vorzugsweise aus einem sterilisierbaren Metall hergestellte Extraktionsvorrichtung 1 zum Entfernen transvenös implantierter Herzschrittmacher- oder Defibrillator-Elektroden, die mit ihrem distalen Ende im Herzmuskel eines Patienten verankert sind. Solche Herzschrittmacher- oder Defibrillator-Elektroden sind über ein Elektrodenkabel mit einem zugeordneten Herzschrittmacher bzw. Defibrillator verbunden.

Die Extraktionsvorrichtung 1 verfügt über ein Verankerungsteil 4, einen Steuerdraht 8 und ein in der Zeichnung stark verkürzt dargestelltes Rohr 11 mit einer Länge in der Größenordnung von einem halben Meter bis einem Meter. Das Rohr 11 hat einen Außendurchmesser von weniger als einem Millimeter, beispielsweise 0,4 bis 0,8 Millimeter. Der Steuerdraht 8 hat einen Durchmesser von weniger als 0,5 Millimeter, beispielsweise 0,2 Millimeter und ist im Innern des Rohres 11 leicht gleitend geführt.

Das Verankerungsteil 4 besteht bei dem in Fig. 1 dargestellten Ausführungsbeispiel aus dem am distalen Ende des Rohres 11 ausgebildeten Rohrabschnitt 12.

In einem bevorzugten Ausführungsbeispiel der Erfindung ist die Länge des Verankerungsteils wesentlich kürzer als die Länge des ein längliches Innenlumen aufweisenden Gegenstandes. Jedoch ist es auch möglich, eine größere Länge für das Verankerungsteil vorzusehen. Insbesondere kann die Länge des Verankerungsteils 4 der Länge des das Innenlumen aufweisenden Gegenstands entsprechen oder sogar größer sein.

Der Rohrabschnitt 12 verfügt bei dem in Fig. 1 dargestellten Ausführungsbeispiel über sechs Wandausschnitte 13. Die zwischen den Wandausschnitten 13 verbleibenden Rohrsegmente bilden Führungsringe 14, zwischen denen sich rinnenförmige Stege 15 erstrecken. Die rinnenförmigen Stege 15 liegen sich bei dem in Fig. 1 dargestellten Ausführungsbeispiel abwechselnd gegenüber und sind im axialen Abstand voneinander angeordnet. Ihre Länge beträgt ein Mehrfaches des Durchmessers des Rohres 11, beispielsweise das Dreifache des Rohrdurchmessers.

Der sich durch die Führungsringe 14 entlang den rinnenförmigen Stegen 15 durch das Verankerungsteil 4 und das Rohr 11 erstreckende Steuerdraht 8 ist bei dem in Fig. 1 dargestellten Ausführungsbeispiel der Erfindung an seinem distalen Ende 16 mit dem distalen Ende 17 des Verankerungsteils 4 fest verbunden, jedoch gegenüber den Führungsringen 14 und den rinnenförmigen Stegen 15 frei beweglich. Aus diesem Grunde kann durch einen Zug am Steuerdraht 8 bei feststehendem Rohr 11 durch Stauchen des Verankerungsteils 4 eine Verformung des Verankerungsteils 4 in eine Verriegelungsgestalt bewirkt werden.

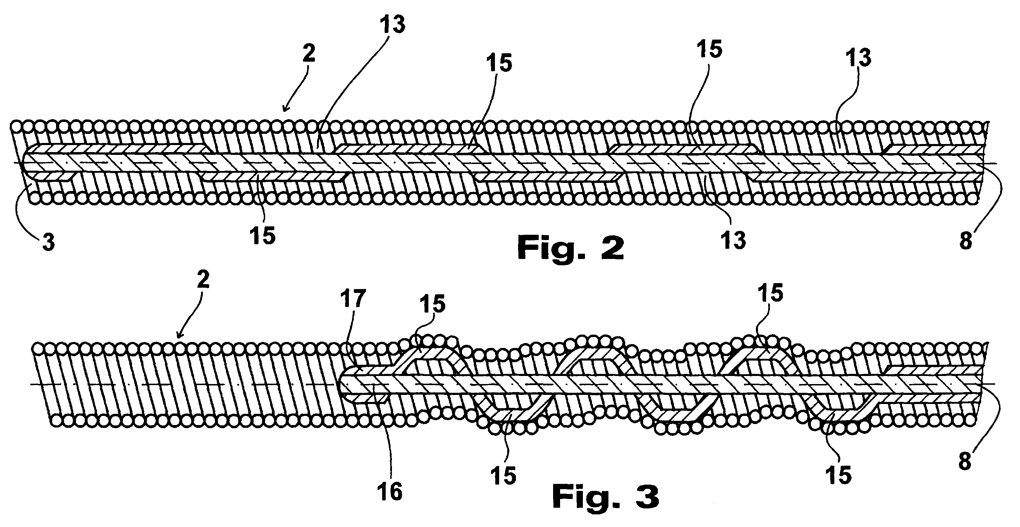

Zur Erzeugung der Verformung ist es erforderlich, auf das Verankerungsteil 4 mit Hilfe des Steuerdrahtes 8 eine axiale Kraft auszuüben. Die hierfür vorgesehene und in Fig. 1 rechts dargestellte Betätigungseinrichtung 20 besteht aus einem Handgriff 21 zur Aufnahme des Rohres 11 und einer Betätigungshülse 22, die es gestattet, mit Hilfe einer Gewindeanordnung eine Rotation mit einer Drehkraft in eine Zugkraft auf den Steuerdraht 8 umzusetzen, um das Verankerungsteil 4 nach dem Einführen in das distale Ende der in Fig. 2 dargestellten Spiralwendel 2 des Herzschrittmacher- oder Defibrillator-Elektrodenkabels zu aktivieren und zu verankern.

Das Rohr 11 erstreckt sich durch eine Rohrbefestigungshülse 23, die ihrerseits in einem Griffkopf 24 befestigt ist. Der Griffkopf 24 ist am distalen Ende einer Griffhülse 25 befestigt. Das proximale Ende des Rohres 11 sowie der Steuerdraht 8 erstrecken sich durch die am Griffkopf 24 befestigte Rohrbefestigungshülse 23. Der Steuerdraht 8 verläuft bis zu einer Drahtbefestigungshülse 26, die mit dem proximalen Ende des Steuerdrahtes 8 fest verbunden und ihrerseits an einem Verbindungsstück 33 befestigt ist.

Das Verbindungsstück 33 ist im Innern der Griffhülse 25 zusammen mit dem Steuerdraht 8 axial verschiebbar und mit dem ersten Ende einer axial verschiebbaren Verbindungshülse 27 fest verbunden. Die Verbindungshülse 27 ist in der Griffhülse 25 axial verschiebbar geführt. Als Drehsperre verfügt die Verbindungshülse 27 über einen in Fig. 1 erkennbaren Längsschlitz, in den ein Stift 28 hineinragt.

Die in Längsrichtung verschiebbare Verbindungshülse 27 ist an ihrem zweiten Ende mit einem Gewindestift 29 fest verbunden, der aus der Griffhülse 25 des Handgriffs 21 herausragt. Der Gewindestift 29 verfügt an seinem proximalen Ende über ein Verbindungselement 30, das mit einer Querbohrung für ein Zugseil versehen sein kann.

Durch Verdrehen der mit einem Innengewinde versehenen Betätigungshülse 22 auf dem Gewindestift 29 kann die Stirnfläche 31 der Betätigungshülse 22 gegen die Endfläche 32 der Griffhülse 25 angezogen werden, um auf diese Weise sehr feinfühlig eine Zugkraft auf den Gewindestift 29 und damit die Verbindungshülse 27, das Verbindungsstück 33, die Drahtbefestigungshülse 26 und damit den Steuerdraht 8 auszuüben.

Zum Aktivieren der in Fig. 1 dargestellten Extraktionsvorrichtung ist es daher lediglich notwendig, nach Einführung des Verankerungsteils 4 in die in Fig. 2 ohne ihre Ummantelung dargestellte Spiralwendel 2 die Betätigungshülse 22 durch Verdrehen gegen die Griffhülse 25 in Anschlag zu bringen und durch weiteres Drehen ein Verkürzen des Verankerungsteils 4 zu bewirken.

Fig. 2 zeigt das Verankerungsteil 4 nach dem Einführen in die Spiralwendel 2 in seiner Verschiebegestalt.

In Fig. 3 ist in einer der Fig. 2 entsprechenden Darstellung dargestellt, wie durch ein Verkürzen des Verankerungsteils 4 die rinnenförmigen Stege 15 unter Führung durch die Führungsringe 14 radial nach außen verformt werden und dabei gegen die Innenwand der Spiralwendel 2 angedrückt werden. Da auf den den Stegen 15 jeweils gegenüberliegenden Seiten die Spiralwendel 2 keinem Gegendruck ausgesetzt ist, erfolgt im Bereich der rinnenförmigen Stege 15 jeweils ein Spiralversatz, der in Fig. 3 deutlich zu erkennen ist. Die Aufweitungen der rinnenförmigen Stege 15 in axialer Richtung führen somit nicht nur lediglich zu einem Reibschluß des Verankerungsteils 4 mit der Spiralwendel 2 sondern an mehreren Stellen zu einem Spiralversatz und damit einem Formschluß. Bei dem in den Fig. 2 und 3 dargestellten Ausführungsbeispiel sind sechs Wandausschnitte 13 und somit sechs rinnenförmige Stege 15 vorgesehen. Selbstverständlich kann die Zahl der Stege 15 nach unten oder nach oben von der dargestellten Zahl abweichen, wobei bereits mit zwei in axialer Richtung gegeneinander verschobenen rinnenförmigen Stegen 15 der Effekt des Spiralversatzes zur sicheren Befestigung erreicht werden kann.

Infolge der in Fig. 3 erkennbaren mehrfachen Verformung aufgrund einer Zugkraft am Steuerdraht 8 ergibt sich eine sehr zuverlässige Verankerung, die es gestattet, infolge eines Formschlusses auch hohe Kräfte bei Spiralwendeln mit weit unterschiedlichen Durchmessern zu übertragen.

Sollte es in einem ungünstigen Fall nicht möglich sein, das Elektrodenkabel zu entfernen, so ist es möglich, das in Fig. 3 in seiner Verriegelungsgestalt dargestellte Verankerungsteil 4 zu verschlanken und in eine der ursprünglichen Gestalt in Fig. 2 näherungsweise entsprechende Verschiebegestalt zu überführen, indem bei festgehaltenem Rohr 11 beziehungsweise festgehaltener Betätigungseinrichtung 20 eine in Fig. 3 nach links wirkende Schubkraft auf den Steuerdraht 8 nach Lösen der Betätigungshülse 22 ausgeübt wird. Da der Steuerdraht 8 an seinem distalen Ende 16 mit dem distalen Ende 17 des Verankerungsteils 4 fest verbunden ist, kann beim Vorschieben des Steuerdrahtes 8 nach erfolgter Stauchung des Verankerungsteils 4 eine Zugkraft ausgeübt werden, die das Verankerungsteil 4 axial verlängert und dabei radial verkleinert. Alternativ ist es auch möglich, einfach nach Lösen der Betätigungshülse 22 am Handgriff 21 zu ziehen, so daß bei leicht verformbaren rinnenförmigen Stegen 15 eine Rückverformung des Verankerungsteils 4 auftritt, die ein Herausziehen des sich dann streckenden Verankerungsteils 4 gestattet.

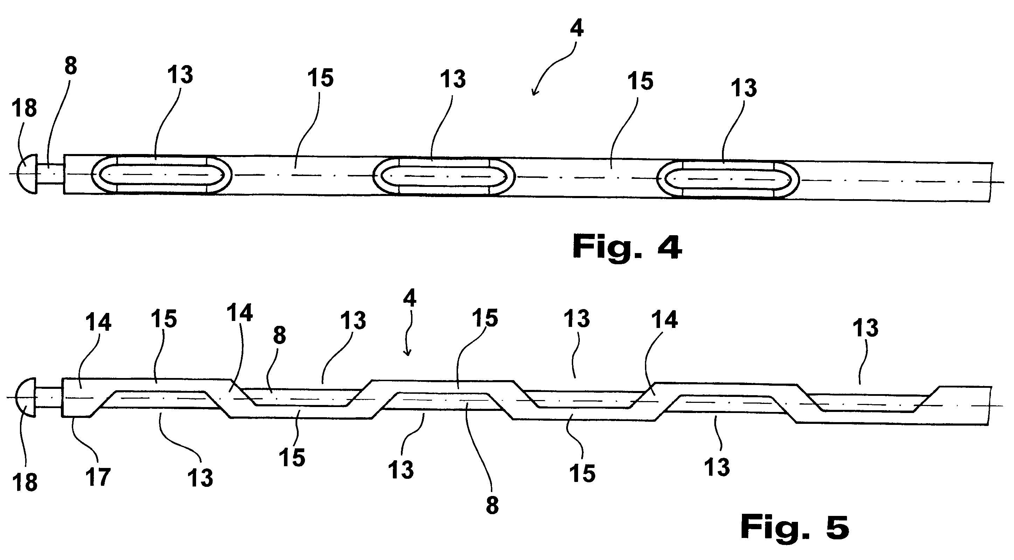

In den Fig. 4 und 5 ist das distale Ende einer Extraktionsvorrichtung 1 mit einer gegenüber dem Ausführungsbeispiel nach den Fig. 1 bis 3 veränderten Kraftübertragung zwischen dem distalen Ende des Steuerdrahtes 8 und dem distalen Ende 17 des Verankerungsteils 4 dargestellt.

Wie man in den Fig. 4 und 5 erkennt, durchquert der Steuerdraht 8 das Verankerungsteil 4 und ist in diesem axial beweglich durch die Führungsringe 14 geführt, die jeweils über rinnenförmige Stege 15 miteinander verbunden sind. Der am distalen Ende 17 des Verankerungsteils 4 ausgebildete Führungsring 14 dient bei dem in den Fig. 4 und 5 dargestellten Ausführungsbeispiel als Anschlag für einen Anschlagkopf 18, der am distalen Ende des Steuerdrahtes 8 ausgebildet ist. Der Anschlagkopf 18 gestattet es, auf das Verankerungsteil 4 eine Zugkraft zum Verformen des Verankerungsteils 4 auszuüben, durch die das Verankerungsteil 4 in die Verriegelungsgestalt entsprechend der Fig. 3 überführt werden kann. Zum Entriegeln kann zwar keine Zugkraft mit Hilfe des Anschlagkopfes 18 ausgeübt werden, jedoch kann in der vorbeschriebenen Weise nach Entriegeln der Betätigungseinrichtung eine Rückverformung des Verankerungsteils 4 durch einfaches Herausziehen erreicht werden.

Wie man in den Fig. 4 und 5 erkennt, verfügt das Verankerungsteil 4 auf seiner in Fig. 4 gezeigten Oberseite über drei im axialen Abstand voneinander ausgebildete Wandausschnitte 13. Auf der gegenüberliegenden Unterseite des Verankerungsteils 4 befinden sich ebenfalls drei im axialen Abstand voneinander angeordnete Wandausschnitte 13, wobei die Anordnung regelmäßig getroffen worden ist, indem zwischen benachbarten Wandausschnitten 13 jeweils die gleichen Abstände und jeweils der gleiche Versatz von 180 Grad vorgesehen sind. Statt der gegenüberliegenden und regelmäßigen Anordnung ist es auch möglich, die Wandausschnitte 13 in einem Winkel gegeneinander versetzt anzuordnen und/oder statt einer periodischen Versetzung eine unregelmäßige Versetzung vorzunehmen.

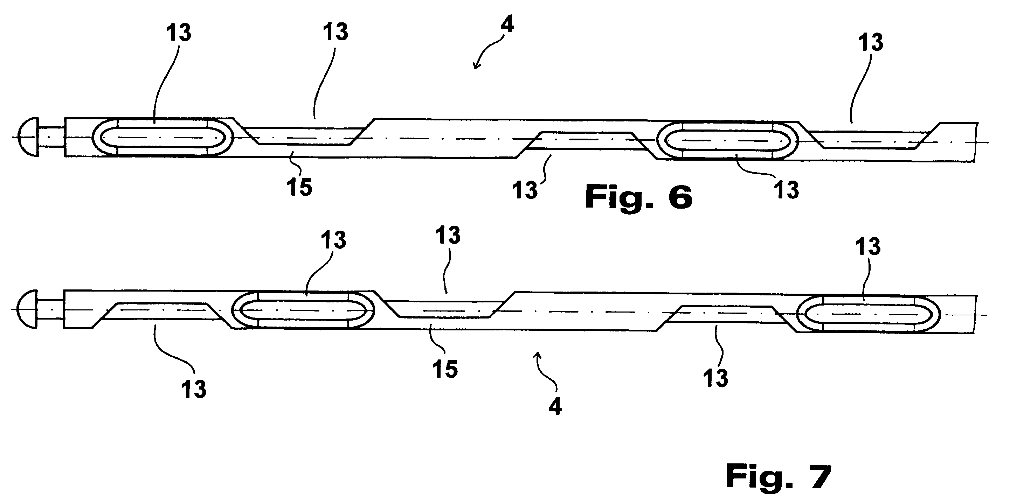

Die Fig. 6 und 7 zeigen ein Verankerungsteil 4, bei dem benachbarte Wandausschnitte 13 nicht um 180 Grad sondern jeweils um 90 Grad in Umfangsrichtung gegeneinander versetzt angeordnet sind. Andere Winkel als die Winkel von 180 Grad und 90 Grad sind ebenfalls möglich.

Man erkennt in den Fig. 4 bis 7, daß die Wandausschnitte 13 an ihren vorderen und hinteren Enden eine Anschrägung von etwa 45 Grad aufweisen. Selbstverständlich können auch Anschrägungen mit einem größeren oder kleineren Winkel verwendet werden. Außerdem erkennt man, daß die Wandausschnitte 13 im mittleren Bereich eine konstante Tiefe aufweisen, wodurch sich in diesem Bereich rinnenförmige Stege 15 mit konstanter Querschnittsform bilden. Je tiefer die Wandausschnitte 13 in den Rohrmantel hineinragen, um so geringer wird die Höhe der rinnenförmigen Stege 15 und damit die erforderliche Kraft zur Verformung des Verankerungsteils 4 zwecks Verriegelung.

Die beschriebenen Abwandlungen des Verankerungsteils 4 verfügen jeweils über rinnenförmige Stege 15, denen in Umfangsrichtung und insbesondere gegenüberliegend keine weiteren Stege zugeordnet sind. Bei einem in der Zeichnung nicht dargestellten Ausführungsbeispiel sind die Wandausschnitte 13 durch Wandeinschnitte mit verringerter Breite (im Extremfall nur ein Schlitz) gebildet, so daß in Umfangsrichtung mehrere rinnenförmige Stege vorhanden sind. Auch bei einer derartigen Anordnung erfolgt aufgrund der in axialer Richtung gegeneinander versetzten radialen Aufweitungen infolge eines Spiralversatzes in der Spiralwendel 2 des Elektrodenkabels ein Formschluß, der das Ausüben einer wesentlich höheren Auszugskraft gestattet, als dies dann der Fall ist, wenn lediglich in Umfangsrichtung verteilte Aufweitungen vorliegen, denen in Längsrichtung keine einen Spiralversatz erzeugende Aufweitungen zugeordnet sind.

## Patentansprüche

1. Vorrichtung zum Herausziehen eines ein längliches Innenlumen aufweisenden Gegenstandes aus seiner Verankerung in einem Körper, insbesondere einer Herzschrittmacher- oder Defibrillator-Elektrode, mit einem eine Spiralwendel aufweisenden Elektrodenkabel, wobei die Vorrichtung ein in das Innenlumen des Gegenstandes einführbares Verankerungsteil, das mit Hilfe einer Betätigungseinrichtung aktivierbar ist, und ein Rohr aufweist, durch das sich ein mit der Betätigungseinrichtung verbundener Steuerdraht erstreckt, durch den bei einer Relativbewegung zwischen dem Rohr und dem Steuerdraht auf das Verankerungsteil eine axial einwirkende Kraft einleitbar ist, durch die das Verankerungsteil zwischen einer Verschiebegestalt und einer aufgeweiteten Verriegelungsgestalt verformbar ist, **dadurch gekennzeichnet**, daß das Verankerungsteil (4) in seiner Verriegelungsgestalt über mehrere in axialer Richtung gegeneinander versetzte radiale Aufweitungen (15) verfügt.

2. Vorrichtung nach Anspruch 1, dadurch gekennzeichnet, daß das Verankerungsteil (4) ein Rohrabschnitt (12) ist, der mit sich axial erstreckenden Wandeinschnitten (13) versehen ist, die in axialem Abstand (15) voneinander angeordnet sind.

3. Vorrichtung nach Anspruch 2, dadurch gekennzeichnet, daß die Wandeinschnitte (13) abwechselnd auf sich radial gegenüberliegenden Seiten der Rohrwandung (12) ausgebildet sind.

4. Vorrichtung nach Anspruch 2, dadurch gekennzeichnet, daß benachbarte Wandeinschnitte (13) in Umfangsrichtung des Rohrumfangs versetzt sind.

5. Vorrichtung nach einem der Ansprüche 1 bis 4, dadurch gekennzeichnet, daß zwischen benachbarten Wandeinschnitten (13) in radialer Richtung leicht verformbare Stege (15) verbleiben.

6. Vorrichtung nach einem der Ansprüche 1 bis 5, dadurch gekennzeichnet, daß die Länge der Wandeinschnitte (13) ein Mehrfaches, insbesondere etwa das Dreifache, des Rohrdurchmessers (12) beträgt.

7. Vorrichtung nach einem der Ansprüche 1 bis 6, dadurch gekennzeichnet, daß die Wandeinschnitte als Wandausschnitt (13) ausgebildet sind.

8. Vorrichtung nach Anspruch 7, dadurch gekennzeichnet, daß im mittleren Bereich der Wandausschnitte (13) die Rohrwandung bis auf eine Rinnenform (15) abgetragen ist.

9. Vorrichtung nach Anspruch 8, dadurch gekennzeichnet, daß die Wandausschnitte (13) an ihren axialen Enden mit der Rohrlängsachse einen Winkel von etwa 30 Grad bis 90 Grad bilden.

10. Vorrichtung nach einem der Ansprüche 1 bis 9, dadurch gekennzeichnet, daß das distale Ende (16) des Steuerdrahtes (8) fest mit dem distalen Ende (17) des Rohrabschnitts (4, 12) verbunden ist.

11. Vorrichtung nach einem der Ansprüche 1 bis 9, dadurch gekennzeichnet, daß das distale Ende (16) des Steuerdrahtes (8) einen Anschlagkopf (18) aufweist, der gegen das distale Ende (17) des Rohrabschnittes (4, 12) anschlagbar ist.

12. Vorrichtung nach einem der Ansprüche 1 bis 11, dadurch gekennzeichnet, daß das distale Ende des Rohres (11) als verformbarer Rohrabschnitt (4, 12) ausgebildet ist.

13. Vorrichtung nach einem der Ansprüche 1 bis 11, dadurch gekennzeichnet, daß das Verankerungsteil (4, 12) vom Rohr (11) getrennt beziehungsweise trennbar ist.

14. Vorrichtung nach einem der Ansprüche 1 bis 13, dadurch gekennzeichnet, daß das Verankerungsteil (4) eine Länge aufweist, die kürzer als die Länge des das längliche Innenlumen aufweisenden Gegenstandes (2) ist.

15. Vorrichtung nach einem der Ansprüche 1 bis 13, dadurch gekennzeichnet, daß der Verankerungsteil (4) eine Länge aufweist, die in etwa der Länge des das Innenlumen aufweisenden Gegenstandes (2) entspricht.

16. Vorrichtung nach einem der Ansprüche 1 bis 13, dadurch gekennzeichnet, daß der Verankerungsteil (4) eine Länge aufweist, die größer als die Länge des das längliche Innenlumen aufweisenden Gegenstandes (2) ist.
